Europäisches Patentamt

European Patent Office

Office européen des brevets

(19)

(11) Publication number: **0 184 260**

**A1**

# EUROPEAN PATENT APPLICATION

(12)

(21) Application number: 85201949.6

(22) Date of filing: 25.11.85

(51) Int. Cl.⁴: **C12Q 1/18**

(30) Priority: 03.12.84 GB 8430483

(43) Date of publication of application:
**11.06.86 Bulletin 86/24**

(84) Designated Contracting States:
**AT BE CH DE FR GB IT LI NL SE**

(71) Applicant: UNILEVER NV
Burgemeester s'Jacobplein 1 P.O. Box 760
NL-3000 DK Rotterdam(NL)
Applicant: UNILEVER PLC
Unilever House Blackfriars P.O. Box 68
London EC4P 4BQ(GB)

(72) Inventor: Bowser, Paul Anthony
Dorset House Latchford Road Gayton
Wirral Merseyside L60 3RW(GB)
Inventor: Evans, Brian
1 Raby Grove Bebington
Wirral(GB)

(74) Representative: Van Gent, Jan Paulus et al
Unilever N.V. Patent Division P.O. Box 137
NL-3130 AC Vlaardingen(NL)

(54) Method and apparatus for detecting microbiological contamination.

(57) The invention pertains to an apparatus and method for detecting microbiological contamination on the basis of measuring pressure increase due to decomposition of hydrogen peroxide by catalase. The invention allows rapid and on-site measurement of microbiological contamination.

EP 0 184 260 A1

# APPARATUS AND METHOD FOR DETECTING MICROBIOLOGICAL CONTAMINATION

The present invention relates to an apparatus and method for detecting microbiological contamination and the assessment of antimicrobial activity. In particular the invention relates to such apparatus whereby the microbiological contamination is determined by measurement of the enzymatic decomposition of hydrogen peroxide.

In a number of industrial areas, such as the dairy food and detergent industries, quality assurance is of a high priority and early detection of microbiological contamination is of the utmost importance.

In general, conventional methods for detecting microbiological contamination require, after processing of the test sample, a period of incubation on culture media for approximately 48 hours before results are available. This may result in high costs in warehouse storage or call-back procedures if contamination is detected. A further drawback of the conventional microbiological detection methods is that they often require technical expertise and special laboratory facilities. Not only do these requirements strongly hinder on-site monitoring, they are also labour-intensive and expensive.

Current methods are normally based on the measurement of a physical property which is directly related to the concentration of micro-organisms or micro-organism released by-products. Examples of such methods are those which measure the impedance, conductance or heat output changes in the microbiological growth medium, or the concentration of ATP via a luminescent reaction. These techniques all require a certain threshold concentration to be reached before detection can occur, and consequently, a microbiological concentration step (culturing) is usually required to ensure sufficient cells for detection. This obviously introduces a delay (approximately 8 hours) into the detection procedure.

In recent years a number of methods for detecting bacterial activity have been proposed which are based on determination by way of catalase measurement. In these methods use is made of the decomposition of hydrogen peroxide by the enzyme catalase which is found in almost all aerobic microbiological systems normally contaminating food and detergent products and industrial and household sites. Catalase rapidly decomposes hydrogen peroxide to oxygen and water, without the activity of the enzyme being very sensitive to the particular reaction conditions.

In the US patent 4,311,794 a catalase method and apparatus is disclosed for direct measurement of bacterial growth activity in the presence and absence of antibiotics. A transducer or other pressure-measuring technique is used to determine the pressure increase in the test tube produced by the catalytic decomposition of peroxide. After the decomposition reaction the test tube is agitated and the increase in pressure is scored on the transducer by perforating the tube stopper with a needle which leads directly into the transducer. Although the period of time for the determination of bacterial activity is considerably reduced, the apparatus of the US patent 4,311,794 is not sufficiently sensitive (without involving an extra time-consuming concentration or culturing step) when only samples of low volume and relatively low bacteria level are available.

It is therefore an object of the present invention to provide an apparatus using the catalase method for the detection of microbiological contamination having improved sensitivity.

It is a further object to provide such apparatus which gives a rapid reading (within one hour) of the microbiological contamination.

It is still a further object to provide such an apparatus which is sufficiently sensitive to detect relatively low levels of microbiological contamination without the need of a culturing or concentration step.

It is still a further object to provide an apparatus which can be applied for the detection of microbiological contamination of both liquid and hard surface samples.

It is still a further object to provide an apparatus which is portable and suitable for in-field monitoring of environmental hygiene.

Surprisingly, it has now been found that when the vessel containing the test sample is connected to a highly sensitive pressure-measuring device by way of a three-way valve and seal system, a considerable improvement in the sensitivity to pressure increases within the vessel due to the release of oxygen in the catalytic decomposition reaction of hydrogen peroxide is achieved.

Accordingly, the present invention provides an apparatus for detecting microbiological contamination, which comprises a pressure-measuring device, and a measuring cell comprising a vessel for receiving quantities of sample material and hydrogen peroxide releasing reagent, means sealing the vessel from atmosphere, and means for connecting the vessel to the pressure-measuring device, characterized in that the pressure-measuring device is suitable for detecting pressure variations in the range from 0.5 to 2000 kg/m², and the means for sealing the vessel comprises a three-way valve unit detachably located in sealing position on the orifice of the vessel, the valve having a first position where the vessel is open to atmosphere and a second position where the vessel is closed to atmosphere and in open connection with the pressure-measuring device.

In a preferred embodiment the apparatus further comprises a second measuring cell. Such dual design of the apparatus enables the simultaneous measurement of a test sample and a control.

Preferably the measuring cell comprises control means for temperature stabilization of the vessel, thereby excluding pressure variations due to temperature fluctuations.

The pressure-measuring device may be of any type suitable for detecting small pressure variations in the range from about 0.5 to 2000 kg/m². The device normally comprises an integrated circuit transducer which converts applied pressure, or differential pressure, into a voltage signal output. This output is then amplified and converted to numerical reading by way of conventional electronic equipment. The electronic equipment in general comprise circuitory conventionally used in electronic control instrumentation of sensitive measuring techniques, such as circuitory for calibration, temperature compensation, control of stability, sensitivity, linearity, etc.

The electronics of the pressure-measuring device is preferably battery-operated to allow for in-field testing without the need of electric mains supply.

The vessel for receiving quantities of sample material and decomposable reagent preferably has a shape and volume which minimize the remaining volume after introduction of the sample material and the reagent. It may be manufactured from any inert, inflexible material such as glass, hard plastics, steel, etc.

The sealing means comprises a three-way valve unit detachably located in sealing position on the orifice of the vessel. In a first position of the valve the vessel is open to atmosphere, whereas in the second position the vessel is closed to atmosphere, but in open connection to the pressure-measuring device.

The sealed vessel and the pressure-measuring device are connected via flexible tubing which is preferably kept as short as possible. In a preferred embodiment the pressure transducer part is mounted on the three-way valve, being electrically connected to the electronic parts of the pressure-measuring device by way of external wiring.

In a further aspect of the present invention a method is provided for detecting microbiological contamination on the basis of the catalase method, comprising the steps of contacting in a suitable vessel a test sample and a concentrated aqueous solution of hydrogen peroxide, subsequently closing said vessel from atmosphere and opening said vessel to a pressure-measuring device suitable for detecting pressure variations in the range of from 0.5 to 2000 $kg/m^2$.

The use of a hydrogen peroxide solution being preferred, if so desired it is also possible to use a solution of a peroxygen compound which under the conditions of the test sample yields hydrogen peroxygen. In general, hydrogen peroxide solutions will be used having concentrations of at least 10% by weight, concentrations of 20-40% by weight being preferred.

The apparatus of the invention and a preferred method for the use thereof will now be described in more detail by way of example and the accompanying drawings illustrating a preferred embodiment of an apparatus according to the present invention, in which:

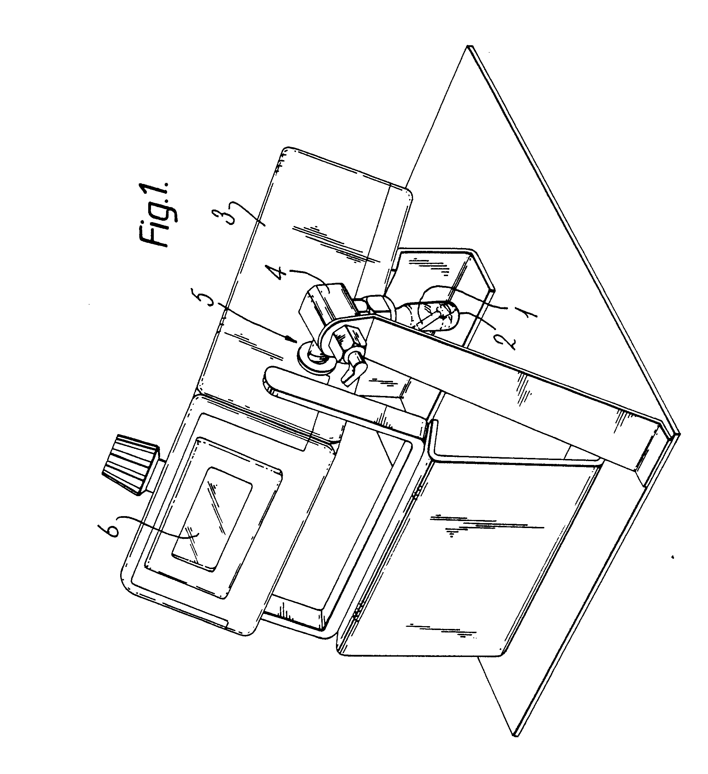

Fig. 1 is a perspective view; and

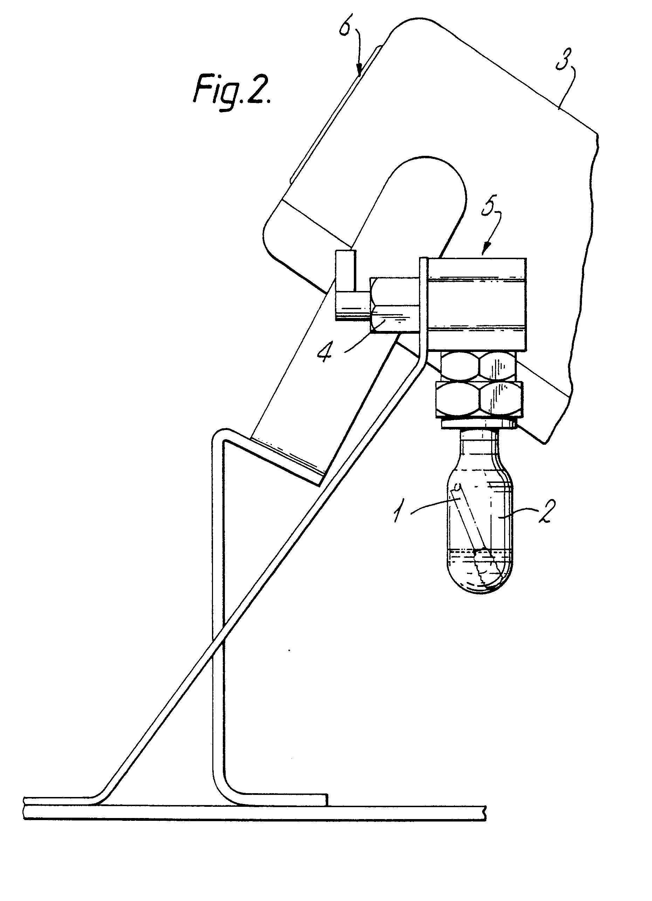

Fig. 2 is an enlarged side view.

EXAMPLE

Twelve hard surface sites were sampled from a domestic bathroom and kitchen, using the following procedure.

Three cotton wool swabs (1) previously moistened with sterile water, were individually wiped over 3 equivalent areas in each site and stored in a sterile plastic vial. The surface area which was wiped, was in each case 100 $cm^2$ (10x10cm). From each site one cotton wool swab was used in a conventional culturing method to determine the total viable count while another served as the control by being autoclaved for 15 minutes at 121°C. The third cotton wool swab was put into a glass vessel (2) of 3 ml volume followed by 0.4 ml of sterile distilled water. Glass vessel (2) was then attached to a pressure-measuring device (3) by way of a three-way valve unit (4) via a screw thread and rubber O-ring connection. Valve (4) was positioned such that the vessel was open to atmosphere. The pressure-measuring device (3) comprised a pressure transducer (5) capable of detecting pressure variations in the range from 0.5 to 2000 $kg/m^2$, and electronic circuitory converting the output signal of transducer (5) to a numerical reading (6). After 10 minutes, the reading of the pressure-measuring device was zeroed, and 25 µl of an aqueous solution of 30% hydrogen peroxide was injected into the water surrounding swab (1) in vessel (2). The vessel was immediately closed from atmosphere by turning valve (4) into the position opening vessel (2) to pressure transducer (5). The reading of the pressure-measuring device was recorded after 10 minutes. The procedure was repeated for the control (autoclaved) swab.

The cotton wool swab from each site for use in the conventional culturing method was transferred to a sterile plastic universal containing 5 mls of one quarter strength Ringers/0.1% Tween 80 as diluent. The sample was vortexed for 30 seconds and serially diluted in 0.1% Peptone/2% Tween 80. One ml amounts of the dilutions were incorporated into Tryptone soya agar using the pour plate technique, and then incubated, aerobically at 28°C for 48 hours.

The results of the pressure readings and the total viable counts are presented in Table I.

These results clearly show the good correlation with the conventional total viable count technique, the method of the present invention taking only up to 20 minutes to get equivalent results, compared to 48 hours for the conventional technique.

Instead of detection of microbiological contamination on hard surfaces, it will be appreciated that the method is equally applicable to contamination detection in liquid and particulate products provided degradation of hydrogen peroxide cannot occur with reactive chemical species, such as e.g. chlorine, in the product to be tested.

TABLE 1

## TABLE 1

| Potentially contaminated site | Pressure reading after 10 mins | | | | bacterial | |
|---|---|---|---|---|---|---|
| | Test | Autoclaved | Difference (X) | Log X | counts (Y) | Log (Y) |
| 1. Kitchen sink | 17000 | 227 | 16773 | 4.23 | $8.60 \times 10^5$ | 5.93 |
| 2. Kitchen sink trap | 12820 | 0 | 12820 | 4.11 | $4.02 \times 10^6$ | 6.60 |
| 3. Kitchen work top | 1251 | 0 | 1251 | 3.10 | $1.99 \times 10^3$ | 3.30 |
| 4. Kitchen floor | 1954 | 238 | 1716 | 3.24 | $4.90 \times 10^4$ | 4.69 |
| 5. Kitchen table top | 1120 | 218 | 902 | 2.96 | $2.70 \times 10^3$ | 3.43 |
| 6. Hob surface | 566 | 0 | 566 | 2.75 | $4.20 \times 10^2$ | 2.62 |
| 7. Inside rim of milk-bottle | 953 | 341 | 612 | 2.79 | $3.20 \times 10^4$ | 4.51 |
| 8. Inside toilet rim* | 140833 | 93 | 140740 | 5.15 | $5.10 \times 10^7$ | 7.71 |
| 9. Side of toilet | 635 | 242 | 393 | 2.59 | $4.90 \times 10^2$ | 2.69 |
| 10. Toilet water after 1 flush | 745 | 178 | 567 | 2.75 | $9.90 \times 10^3$ | 4.00 |
| 11. Nappy bucket* | 38100 | 134 | 37966 | 4.58 | $2.40 \times 10^7$ | 7.38 |
| 12. Wash hand basin (bathroom)* | 46750 | 242 | 46508 | 4.67 | $1.05 \times 10^7$ | 7.02 |

* Pressure meter reading calculated from reducing measurement time (3 or 5 mins).

Claims

1. An apparatus for detecting microbiological contamination, which comprises a pressure-measuring device, and a measuring cell comprising a vessel for receiving quantities of sample material and hydrogen peroxide releasing reagent, means for sealing the vessel from atmosphere, and means for connecting the vessel to the pressure-measuring device, characterized in that the pressure-measuring device is suitable for detecting pressure variations in the range from 0.5 to 2000 kg/m², and the means for sealing vessel comprises a three-way valve unit detachably located in sealing position on the orifice of the vessel, the valve having a first position where the vessel is open to atmosphere and a second position where the vessel is closed to atmosphere and in open connection with the pressure measuring device.

2. An apparatus according to claim 1, comprising means for temperature stabilization of the vessel.

3. An apparatus according to claim 1 or 2, which is battery-operated.

4. An apparatus according to any one of the preceding claims, in which the pressure-measuring device comprises a transducer capable of converting pressure into a voltage signal output.

5. An apparatus according to claim 4, in which the transducer is mounted on the three-way valve.

6. An apparatus according to any one of the preceding claims, comprising two separate measuring cells.

7. A method for detecting microbiological contamination on the basis of the catalase method, comprising the steps of contacting in a suitable vessel a test sample and a concentrated aqueous solution of hydrogen peroxide, subsequently closing said vessel from atmosphere and opening said vessel to a pressure-measuring device suitable for detecting pressure variations in the range of from 0.5 to 2000 kg/m².

8. A method according to claim 7, in which a 20-40% by weight hydrogen peroxide solution is used.

Fig.1.

0 184 260

*Fig.2.*

## European Patent Office

## EUROPEAN SEARCH REPORT

Application number

| | DOCUMENTS CONSIDERED TO BE RELEVANT | | EP 85201949.6 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | WO - A1 - 82/02 561 (WEAVER) <br> * Abstract; pages 1-4; claims * <br> -- | 1 | C 12 Q 1/18 |
| A | WO - A1 - 84/03 903 (SILMAN) <br> * Abstract * <br> -- | 1 | |
| D,A | US - A - 4 311 794 (MELNICK) <br> * Abstract; claims * <br> ---- | 1 | |

| TECHNICAL FIELDS SEARCHED (Int. Cl 4) |
|---|
| C 12 Q 1/00 <br> G 01 N 7/00 <br> G 01 N 33/00 |

The present search report has been drawn up for all claims

| Place of search | Date of completion of the search | Examiner |
|---|---|---|
| VIENNA | 14-02-1986 | ERBER |